# EUROPEAN PATENT APPLICATION

(11) **EP 3 078 357 A1**
(43) Date of publication of application: **12.10.2016**
(21) Application number: 15162801.3
(22) Date of filing: 08.04.2015
(51) Int. Cl.: A61F 6/00

(54) **Packaging for sanitary objects, such as condoms and the like**

(71) Applicant: Nijholt, Hendrik Johannes Karl, 8713 JE Hindeloopen (NL)
(72) Inventor: Nijholt, Hendrik Johannes Karl, 8713 JE Hindeloopen (NL)
(74) Representative: 't Jong, Bastiaan Jacob

(57) **Abstract**

The invention relates to a packaged sanitary object, such as a condom and the like, comprising:
- opposed first and second material layers sealed together along a perimeter and defining a sealed cavity between the opposed layers;
- a sanitary object, such as a condom, arranged in the sealed cavity; and
- a bag arranged in the sealed cavity.

## Description

The invention relates to a packaged sanitary object, such as a condom and the like.

In practice, disposal of a sanitary object, in particular a condom after use still presents several difficulties. The desirability of removing the condom as soon as possible after ejaculation is known, without this necessarily implying termination of the sexual encounter.

Therefore, and at that special moment, the problem arises of what to do to quickly dispose of the used condom, with its unsightly appearance and impregnated with body fluids (semen, vaginal secretions, lubricants, etc.), so that the sexual encounter is minimally interrupted and there is a minimal loss of spontaneity.

A frequent domestic solution consists in placing the used condom on a home-made provisional wrapper or cover, such as a paper tissue or strip of toilet paper of the kind that is normally available in households.

This provisional wrapper or cover, together with the condom, must later be thrown into a domestic waste bin. However, there are several drawbacks to this solution. On the one hand, the use of this provisional wrapper or home-made paper might be disagreeable and distasteful for the user because it interferes with the sexual encounter. On the other hand, the solution is not too hygienic since fluids from the used condom might leak onto the surfaces on which the provisional wrapper or paper with the used condom was placed temporarily. In addition to these drawbacks, disposing of the paper with the used condom by depositing it in the domestic waste bin might cause certain users a sense of shame or lack of privacy in the presence of third parties, who might see the discarded condom in the rubbish. In view of these drawbacks, the chances are high that the used condom will end up being thrown into a toilet instead of into the household waste bin.

Throwing the used condom down the toilet is not an acceptable method of disposal, since discarded condoms can cause problems in the sanitary sewers and, even more seriously, cause environmental pollution problems. The drawbacks associated with disposing of the used condom are frequently exacerbated when sexual intercourse takes place outside the user's habitual residence, in hotels, vehicles, other people's houses or in public spaces such as parks or beaches. In these cases, it is even more likely that the used condom ends up in a toilet, on the street or any other inappropriate place, far from a rubbish container that would allow the condom to be otherwise collected by the municipal service and thrown into a controlled landfill site.

Alternatively to the provisional wrapper home-made paper solution or the like, several devices, cleaning kits or hygiene packs, greater or small in size and complexity, are known, such as described in the patent application US 5638949, disclosing a device to dispose of a used condom, which includes a container that can be closed to form a receptacle for the used condom. However, such a solutions is costly, when supplied for each condom separately.

Accordingly, it is an object of the invention to reduce or even remove the above mentioned disadvantages.

This object is achieved according to the invention with a packaged sanitary object, such as a condom and the like, comprising:
- opposed first and second material layers sealed together along a perimeter and defining a sealed cavity between the opposed layers;
- a sanitary object, such as a condom, arranged in the sealed cavity; and
- a bag arranged in the sealed cavity.

According to the invention, the sanitary object, such as a condom, is packaged between two material layers together with a bag. When the packaging is opened, similar to known packaged condoms, and the condom is taken out of the packaging, the bag will also come out of the packaging.

Typically, the packaging will be left on a bedside table or the like, while sexual intercourse takes place and the condom is used. After use, the condom as well as the material layers can be put quickly in the bag still laying on the bedside table, such that the sexual encounter can continue.

At the end of the sexual encounter, the bag containing the material layers, as well as the used condom (or other sanitary object) can be thrown away in a waste bin. Because the condom is contained in a bag, third parties will not recognize the bag in the rubbish as a used condom.

In a preferred embodiment of the packaged sanitary object the bag is folded at least along a folding line positioned between the opening and the bottom of the bag. In this embodiment, the bag is at least folded double in length. When the bag is taken out of the packaging, the bag can be unfolded such that the bag is sufficiently large to easily put in the used condom and the material layers.

In a further preferred embodiment of the packaged sanitary object according to the invention, the bag is folded at least along a folding line extending between the opening and the bottom of the bag.

In this embodiment, the bag is folded at least once in width direction. Together with at least one fold in length direction, the size of the bag can be reduced to at least a quarter, such that it is easily incorporated in the packaging for the sanitary object, while providing a sufficiently large bag to discard both the used condom as well as the material layers of the packaging.

In another embodiment of the packaged sanitary object according to the invention the bag is folded around the sanitary object.

When the condom or other sanitary object is taken out of the packaging, it is ensured that in this embodiment, at least a part of the bag also comes out of the packaging. This ensures that the user is aware of the presence of a bag to discard the used condom, minimizing the risk that the used condom is discarded via a toilet or the like.

In a further preferred embodiment of the packaged sanitary object according to the invention the bottom of the bag is fixed to the seal sealing together the first and second material layers.

By attaching the bag to the sealed material layers it is ensured that the bag and the material layers will not be separated and that when the sanitary object is discarded using the bag, the material layers will also be discarded in the same way.

Preferably, at least part of the bottom of the bag is sealed together with the first and second material layers. During manufacturing of the packaged sanitary object, the two material layers will be brought together with the sanitary object in between, after which the seal will be made. By ensuring that part of the bag overlaps with the sealing edge, the bag will be fixed to the seal just by sealing the two material layers together.

In yet another embodiment of the packaged sanitary object according to the invention, the peripheral edge of the sealed together first and second material layers comprises at least one cut or recess for initiating a tear for tearing open the packaging.

Providing at least one cut or recess ensures that the user of the packaged sanitary object will open the packaging at a predefined position by tearing the part of the packaging starting at the at least one cut or recess.

Preferably, the at least one cut or recess is arranged on or adjacent to the side opposite of the side to which the bag is fixed. This ensures that the packaging is opened at the side opposite of the attachment of the bag to the seal and that when opening the packaging, the bag is not torn apart.

These and other features of the invention will be elucidated in conjunction with the accompanying drawings.
Figure 1 shows a perspective view of a first embodiment of the packaged sanitary object according to the invention with cut-away portions.
Figure 2 shows the packaged sanitary object in a first mode of use.
Figure 3 shows the packaged sanitary object in a second mode of use.
Figure 4 shows a cross sectional view of the packaged sanitary object of figure 1.
Figure 5 shows a cross sectional view of a second embodiment of the packaged sanitary according to the invention.

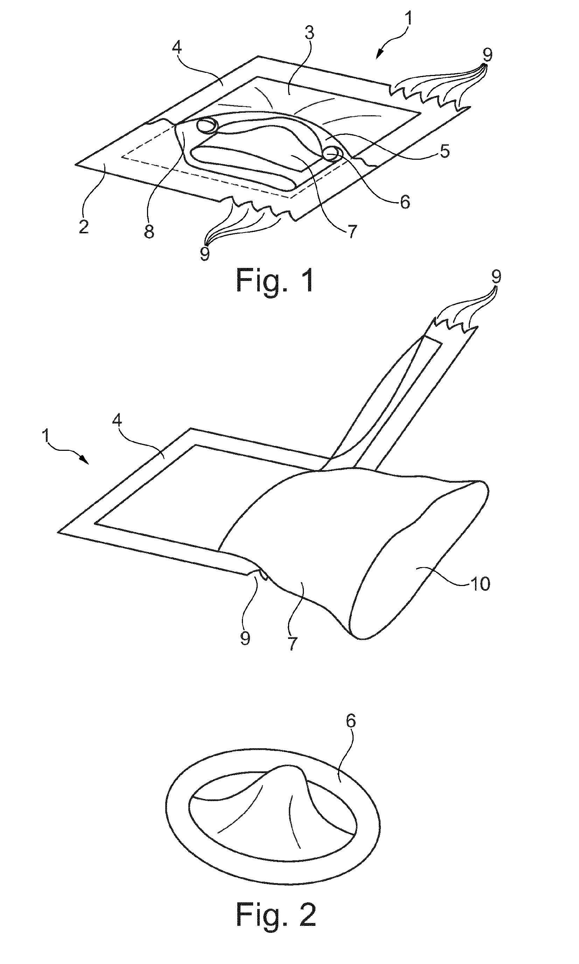

Figure 1 shows a perspective view of a first embodiment 1 of the packaged sanitary object according to the invention with cut-away portions. The packaged sanitary object 1 has a first material layer 2 and a second material layer 3, which are sealed together along a peripheral edge 4.

The sealed first and second material layers 2, 3 define a cavity 5 in which the sanitary object, in particular a condom 6 , and a bag 7 are positioned.

The bag 7 is fixed with its bottom 8 to the seal arranged along the peripheral edge 4. The bag 7 is furthermore folded two times in this embodiment.

The edge of the material layers 2, 3 is provided with recesses 9 adjacent to the side opposite of the side to which the bag 7 is fixed with its bottom 8 to the seal..

Figure 2 shows the packaged sanitary object 1 in a first mode of use. The packaged sanitary object 1 is opened by tearing open the packaging along a recess 9. This allows the user to take out the condom 6, which will also reveal to the user the bag 7, such that the user is reminded on how to discard the condom 6 after use in a discrete and environmental safe way.

Preferably, the bag 7 is also folded along folding lines extending from the bottom 8 of the bag to the opening 10 of the bag 7, such that a sufficient large bag is revealed from the packaging.

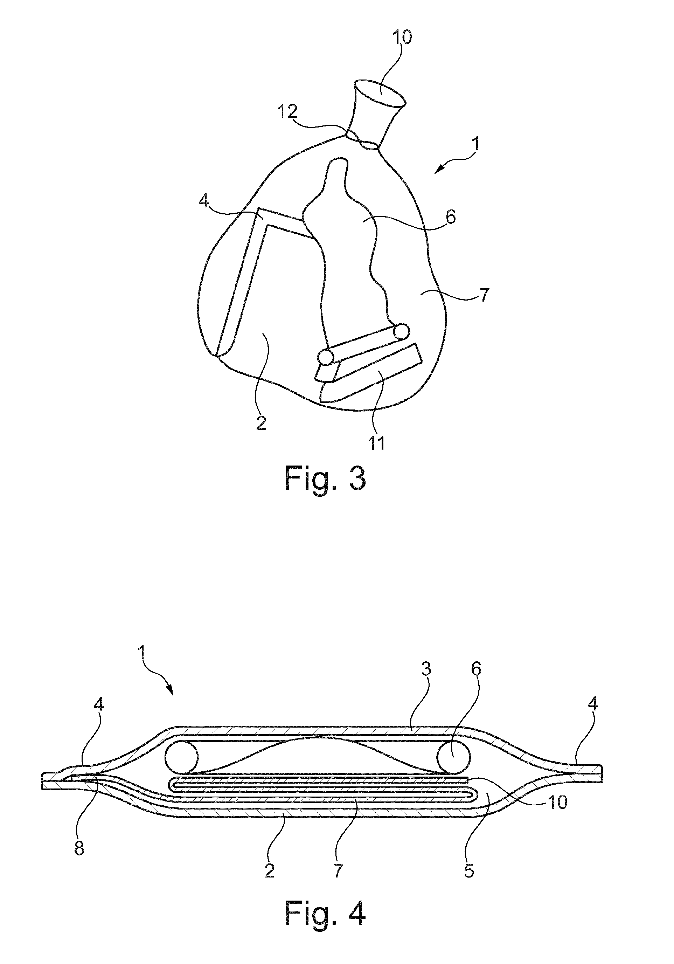

Figure 3 shows the packaged sanitary object 1 in a second mode of use. After use of the condom 6, the bag is wrapped around the packaging 2, 3 and the torn away part 11. The used condom 6 is disposed in the bag 7 via the opening 10, after which a knot 12 is provided near the opening 10 to close the bag 7.

Figure 4 shows a cross sectional view of the packaged sanitary object 1 of figure 1, which clearly shows that the material layers 2, 3 are sealed together along the peripheral edge defining a cavity 5 in which the condom 6 and a double folded bag 7 is arranged. The bottom 8 of the bag 7 is interposed between the edges of the material layers 2, 3, such that after sealing both layers 2, 3 along the peripheral edge 4, the bottom 8 of the bag 7 is fixed to the peripheral edge 4. This ensures that when the packaging is opened, the packaging and the bag 7 are kept together.

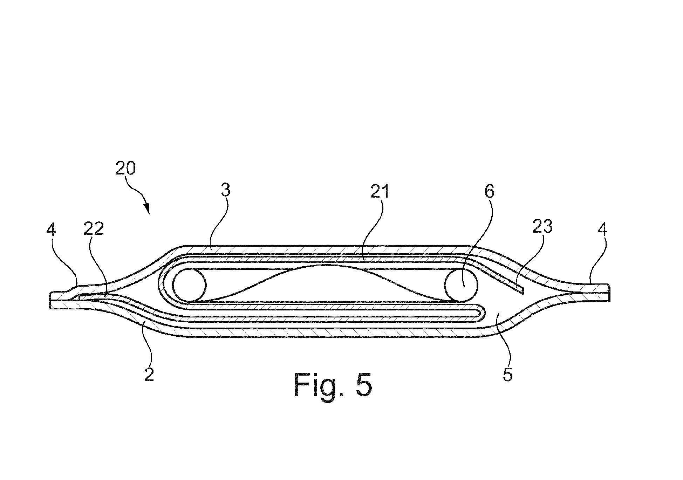

Figure 5 shows a cross sectional view of a second embodiment 20 of the packaged sanitary according to the invention. This embodiment 20 is similar to the embodiment 1 of figure 1 and the same parts are designated with the same reference signs.

Also with this embodiment 20, the material layers 2, 3 are sealed together along the peripheral edge 4 to define a cavity in which a condom 6 and a bag 21 are positioned.

The bag 21 is fixed with the bottom 22 of the bag 21 between and sealed together with the peripheral edges of the material layers 2, 3. The bag 21 then runs from the bottom 22 over the bottom of the cavity 5 to the opposite side, is folded back underneath the condom 6 and finally folded over the condom 6, such that the opening 23 of the bag 21 is positioned on the opposite side of the packaging 20 as the bottom 22 of the bag 21.

When the packaging 20 is opened and the condom 6 is taken out, the bag 21 will almost certainly come out also, as the condom 6 is positioned between the folded bag 21. This ensures that a user will always be pointed to the availability of a discrete way to discard the used condom.

## Claims

1. Packaged sanitary object, such as a condom and the like, comprising:
- opposed first and second material layers sealed together along a perimeter and defining a sealed cavity between the opposed layers;
- a sanitary object, such as a condom, arranged in the sealed cavity; and
- a bag arranged in the sealed cavity.

2. Packaged sanitary object according to claim 1, wherein the bag is folded at least along a folding line positioned between the opening and the bottom of the bag.

3. Packaged sanitary object according to claim 1 or 2, wherein the bag is folded at least along a folding line extending between the opening and the bottom of the bag.

4. Packaged sanitary object according to claim 2 or 3, wherein the bag is folded around the sanitary object.

5. Packaged sanitary object according to any of the preceding claims, wherein the bottom of the bag is fixed to the seal sealing together the first and second material layers.

6. Packaged sanitary object according to claim 5, wherein at least part of the bottom of the bag is sealed together with the first and second material layers.

7. Packaged sanitary object according to any of the preceding claims, wherein the peripheral edge of the sealed together first and second material layers comprises at least one cut or recess for initiating a tear for tearing open the packaging.

8. Packaged sanitary object according to claim 7 and claim 5 or 6, wherein the at least one cut or recess is arranged on or adjacent to the side opposite of the side to which the bag is fixed.
